# EUROPÄISCHE PATENTSCHRIFT

(11) **EP 2 792 292 B1**
(45) Veröffentlichungstag und Bekanntmachung des Hinweises auf die Patenterteilung: **12.09.2018**
(21) Anmeldenummer: 14162728.1
(22) Anmeldetag: 31.03.2014
(51) Int. Cl.: A61B 1/00, A61B 1/018, G02B 23/24, A61B 1/12, A61B 1/06

(54) **Endoskop mit einem starren gebogenen Schaft sowie Verfahren zur Herstellung eines solchen Endoskops**
Endoscope with a rigid bent shaft and method for manufacturing such an endoscope
Endoscope doté d'une tige fixe courbée et procédé de fabrication d'un tel endoscope

(30) Priorität: 19.04.2013 DE 102013207109
(43) Veröffentlichungstag der Anmeldung: 22.10.2014
(73) Patentinhaber: Henke-Sass, Wolf GmbH, 78532 Tuttlingen (DE)
(72) Erfinder: Häckl, Norbert, 88637 Leibertingen (DE); Rapp, Stefan, 78050 Villingen-Schwenningen (DE)
(74) Vertreter: Patentanwälte Geyer, Fehners & Partner mbB

(56) Entgegenhaltungen:
- DE-A1-102007 032 200
- DE-A1-102009 022 118
- GB-A- 1 078 036
- JP-A- H09 201 325
- US-A- 4 606 330
- US-A1- 2013 046 139

## Beschreibung

Die vorliegende Erfindung betrifft ein Endoskop mit einem starren gebogenen Schaft sowie ein Verfahren zur Herstellung eines solchen Endoskopes.
Solche Endoskope sind z. B. aus JP 09-201325 A bekannt und werden beispielsweise im medizinischen Bereich eingesetzt, zum z.B. Untersuchungen und gegebenenfalls Behandlungen im Bereich der Nase durchzuführen. Dazu weisen solche Endoskope in der Regel ein gebogenes Instrumentenrohr mit einem offenen distalen Ende sowie ein entsprechend gebogenes Optikrohr auf. Über das Optikrohr wird ein Bild des entsprechenden Bereichs vor dem distalen Ende aufgenommen und über das Instrumentenrohr kann ein Instrument an der entsprechenden Stelle positioniert werden, um beispielsweise Gewebe zu entfernen. Das Optikrohr und das Instrumentenrohr sind miteinander verbunden und können mehrteilig sein, so daß der durch die beiden Rohre gebildete starre Schaft keine glatte äußere Oberfläche aufweist, sondern Vertiefungen und Kanten. Dies ist nachteilig, da sich dort Verschmutzungen ansammeln können, so daß eine Reinigung und Sterilisation des Endoskopes schwierig ist.

Ausgehend hiervon ist es Aufgabe der Erfindung ein Endoskop mit einem starren gebogenen Schaft bereitzustellen, das ein Instrumentenrohr zur Aufnahme eines Instrumentes sowie ein Optikmodul zur Aufnahme eines Bildes eines Bereiches vor dem distalen Ende des Schaftes aufweist und das leicht gereinigt werden kann.

Erfindungsgemäß wird die Aufgabe durch ein Endoskop mit einem Hauptkörper und einen sich vom Hauptkörper erstreckenden starren Schaft, der einen sich geradlinig erstreckenden ersten Abschnitt, einen daran anschließenden gebogenen zweiten Abschnitt und einen sich an den zweiten Abschnitt anschließenden dritten Abschnitt, der ein distales Ende des Schaftes bildet, aufweist, gelöst, wobei der Schaft zur Aufnahme eines Instrumentes ein einstückig ausgebildetes Instrumentenrohr, das sich bis zum distalen Ende des Schaftes erstreckt und dort ein offenes Ende aufweist, und ein Optikmodul, mittels dem ein Bild eines Bereiches vor dem distalen Ende des Schaftes aufnehmbar ist, aufweist, und wobei ein sich vom Hauptkörper bis zum distalen Ende erstreckendes mehrteiliges Hüllrohr vorgesehen ist, in dem das Instrumentenrohr und das Optikmodul angeordnet sind und das einen geradlinigen Teil für den ersten Abschnitt und einen mit dem geradlinigen Teil verbundenen gebogenen Teil für den zweiten Abschnitt aufweist.
Durch das Hüllrohr kann eine glatte äußere Oberfläche des Schaftes bereitgestellt werden, an der sich einerseits Verschmutzungen nicht so ansammeln und die andererseits leicht zu reinigen ist. Da das Hüllrohr mehrteilig ausgebildet ist, läßt sich das erfindungsgemäße Endoskop auch leicht herstellen. Insbesondere kann das bereits gebogene Instrumentenrohr mit seinem gebogenen Abschnitt in den gebogenen Teil eingeführt werden und ein geradliniger Abschnitt des Instrumentenrohrs in den geradlinigen Teil eingeführt werden. Danach können die beiden Teile miteinander verbunden werden, so daß das gewünschte Hüllrohr vorliegt.
Das Instrumentenrohr steht bevorzugt nicht aus dem distalen Ende des Hüllrohrs vor. Insbesondere kann das distale Ende des Instrumentenrohrs mit dem distalen Ende des Hüllrohrs fluchten.
Ferner ist das Optikmodul innerhalb des Hüllrohrs und steht bevorzugt nicht über das distale Ende vor.
Das von dem geradlinigen Teil abgewandte Ende des gebogenen Teils kann das distale Ende des Schaftes bilden. Alternativ ist es möglich, daß das Hüllrohr einen dritten Teil aufweist, der das distale Ende des Schaftes bildet und mit dem gebogenen Teil verbunden ist. Der dritte Teil kann insbesondere durch spanende Bearbeitung aus einem Vollmaterial hergestellt sein, wohingegen der erste und zweite Teil bevorzugt aus einem Hohlrohr, insbesondere einem gezogenen Hohlrohr, hergestellt sein können. Die das Hüllrohr bildenden Teile sind bevorzugt aus Edelstahl hergestellt.
Die miteinander verbundenen Teile können miteinander verschweißt sein. Bevorzugt werden die Schweißstellen danach geschliffen, so daß das Hüllrohr eine glatte durchgehende äußere Oberfläche aufweist.
Das dritte Teil kann einstückig ausgebildet sein und eine Endplatte aufweisen, die das vom gebogenen Teil abgewandte Ende verschließt, wobei in der Endplatte eine Öffnung für das Instrumentenrohr und zumindest eine Öffnung für das Optikmodul vorgesehen sind.

Die zumindest eine Öffnung für das Optikmodul in der Endplatte kann mittels einer transparenten Scheibe bzw. Platte verschlossen sein. Insbesondere kann es sich um eine Glasplatte bzw. Glasscheibe handeln (beispielsweise Saphirglas). Das Glas kann verlötet sein, um eine hermetische Abdichtung der Öffnung bereitzustellen.

Der gebogene Teil kann eine Biegung von größer als 0° und kleiner als 120°, insbesondere eine Biegung von größer oder gleich 10° und kleiner oder gleich 110° aufweisen. Ferner kann die Biegung im Bereich von 10° bis 100°, 10° bis 90°, 20° bis 120°, 30° bis 120° oder 45° bis 120° liegen.

Das Optikmodul kann insbesondere am distalen Ende im Schaft angeordnet sein. Das Optikmodul kann zumindest eine Abbildungsoptik (z. B. Objektiv) aufweisen. Ferner kann das Optikmodul einen direkt hinter der Abbildungsoptik angeordneten Bildsensor, wie z.B. einen CMOS- oder CCD-Sensor aufweisen. Alternativ kann der Abbildungsoptik eine Übertragungsoptik nachgeordnet sein, die das aufgenommene Bild bis in den Hauptkörper überträgt. Im Hauptkörper kann ein Bildsensor angeordnet sein, um das übertragene Bild aufzunehmen. Alternativ oder zusätzlich kann ein optischer Einblick am Hauptkörper bereitgestellt sein, über den ein Benutzer das in den Hauptkörper übertragene Bild mit seinem Auge wahrnehmen kann.

Ferner kann das erfindungsgemäße Endoskop eine Beleuchtung aufweisen, die über das distale Ende den aufnehmbaren Bereich beleuchtet. Die Beleuchtung kann beispielsweise eine Lichtquelle am distalen Ende aufweisen. Alternativ, kann die Lichtquelle im Hauptkörper angeordnet sein. Zur Übertragung des Lichtes der Lichtquelle kann beispielsweise ein Lichtleiter eingesetzt werden, der dann vom Hauptteil über den Schaft bis zum distalen Ende verläuft. Bei der Lichtquelle kann es sich insbesondere um eine Leuchtdiode oder Laserdiode handeln. Die Lichtquelle gibt bevorzugt Licht im sichtbaren Spektralbereich ab. Alternativ oder zusätzlich kann sie auch Licht in anderen Wellenlängenbereichen abgeben, wie z.B. im Infrarotbereich.

Ferner ist es möglich, daß das Endoskop selbst keine Lichtquelle aufweist, sondern nur einen Lichtleiteranschluß am Hauptkörper, über den dann Licht einer externen Lichtquelle bis zum distalen Ende geführt werden kann.

Das Instrumentenrohr weist bevorzugt eine Querschnittsform auf, die neben einem kreisförmigen Querschnittsbereich noch einen weiteren Bereich umfaßt. Dieser weitere Bereich kann dann bei eingesetztem Instrument, das den kreisförmigen Querschnittsbereich belegt, als Spül- und/oder Absaugkanal genutzt werden. Insbesondere kann das Instrumentenrohr einen D-förmigen Querschnitt aufweisen.

Das Hüllrohr kann eine längliche Querschnittsform aufweisen, die zwei gegenüberliegende abgerundete Enden sowie zwei geradlinig erstreckende, die Enden verbindende Seiten aufweist. Die abgerundeten Enden können insbesondere eine Rundung mit konstantem Radius und insbesondere eine halbkreisförmige Rundung aufweisen. Die sich geradlinig erstreckenden Seiten können insbesondere zueinander parallel sein.

Wie bereits ausgeführt, kann im Hauptkörper eine Lichtquelle angeordnet sein, deren Licht über ein Lichtleitsystem bis zum distalen Ende geführt wird, um den vor dem distalen Ende aufnehmbaren Bereich zu beleuchten.

Die Lichtquelle kann in direktem mechanischen Kontakt mit einem ersten Wärmeleitkörper stehen, der von der Lichtquelle erzeugte Wärme zu einer Gehäusewandung des Hauptkörpers leitet, wobei die Wärmeleitfähigkeit des ersten Wärmeleitkörpers größer ist als die der Gehäusewandung. So kann die Gehäusewandung beispielsweise aus Edelstahl und der erste Wärmeleitkörper aus Aluminium hergestellt sein.

Bei dem erfindungsgemäßen Endoskop kann im Hauptkörper zumindest ein weiterer Wärmeleitkörper angeordnet sein, der in thermischem Kontakt mit dem ersten Wärmeleitkörper steht und mit einer Kraft beaufschlagt ist, die ihn gegen die Innenseite der Gehäusewandung drückt. Insbesondere können zwei weitere Wärmeleitkörper angeordnet sein, die einander gegenüberliegen. Die beiden Wärmeleitkörper können voneinander weggedrückt werden. Dazu kann beispielsweise eine Schraube benutzt werden, die zumindest in dem einen der beiden Wärmeleitkörper in einem Innengewinde geführt ist. Das dem Innengewinde abgewandte Ende der Schraube kann gegen den anderen Wärmeleitkörper drücken. Durch entsprechendes Einstellen der Schraube können die beiden Wärmeleitkörper voneinander weggedrückt werden.

Bei dem erfindungsgemäßen Endoskop kann die thermische Kontaktierung des Wärmeleitkörpers mit der Gehäusewandung über eine reine Berührungskontaktierung ohne Wärmeleitkleber oder Wärmeleitpaste erzeugt sein. Alternativ ist es möglich, einen Wärmeleitkleber oder eine Wärmeleitpaste zu verwenden.

Bei dem erfindungsgemäßen Endoskop kann der Hauptkörper sowie der Schaft mit Ausnahme des Instrumentenrohrs gegenüber der Umgebung hermetisch abgedichtet und somit autoklavierbar sein. Unter autoklavierbar wird hier insbesondere verstanden, daß das Endoskop einer vorbestimmten Zeitdauer (beispielsweise mehrere Minuten) Wasserdampf (insbesondere gesättigtem Wasserdampf) von mindestens 100°C bzw. mindestens 130°C zur Sterilisation ausgesetzt wird, ohne daß dabei das Endoskop beschädigt wird (ohne daß insbesondere Wasserdampf in den Schaft (mit Ausnahme des Instrumentenrohrs) und in den Hauptkörper eindringen kann).

Die Aufgabe wird ferner durch ein Verfahren zur Herstellung eines erfindungsgemäßen Endoskopes gelöst, bei dem folgende Schritte ausgeführt werden:
a) Einführen eines gebogenen Abschnittes des Instrumentenrohrs in den gebogenen Teil,
b) Einführen eines geradlinigen Abschnitts des Instrumentenrohrs in den geradlinigen Teil, und
c) (dauerhaftes) Verbinden der beiden Teile.

Die Schritte a) und b) können in beliebiger Reihenfolge ausgeführt werden. Das Verbinden gemäß Schritt c) kann beispielsweise durch Verschweißen erfolgen.

Das erfindungsgemäße Verfahren kann den Schritt des Biegens des Instrumentenrohrs aufweisen. Ferner kann es den Schritt des Biegens eines geradlinigen Hohlrohrs zur Erzeugung des gebogenen Teils aufweisen.

Das erfindungsgemäße Verfahren kann die in Verbindung mit dem erfindungsgemäßen Endoskop einschließlich seiner Weiterbildungen beschriebenen Verfahrensschritte aufweisen. Ferner kann es die Verfahrensschritte aufweisen, um das erfindungsgemäße Endoskop einschließlich seiner Weiterbildungen herzustellen.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer Ausführungsform des erfindungsgemäßen Endoskops;
- Fig. 2: eine Schnittansicht des Endoskops gemäß Fig. 1;
- Fig. 3: eine vergrößerte Draufsicht des distalen Endes 4 des Schaftes 3 des Endoskops gemäß Fig. 1 und 2;
- Fig. 4: eine vergrößerte Ansicht des Details A in Fig. 2, und
- Fig. 5: eine vergrößerte Schnittansicht entlang der Schnittlinien B-B in Fig. 2.

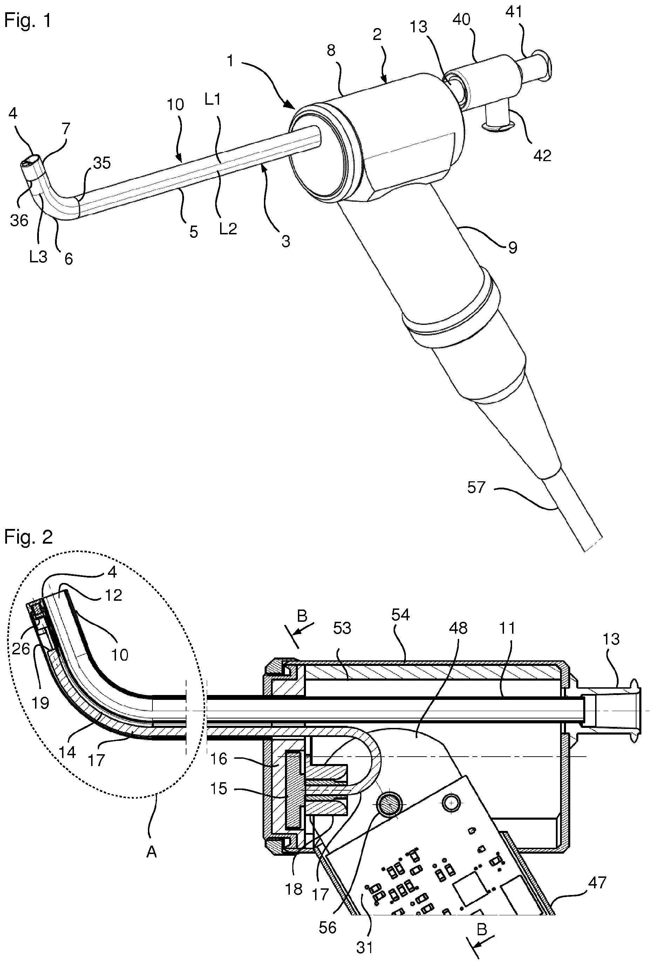

Bei der in Fig. 1 gezeigten Ausführungsform umfaßt das erfindungsgemäße Endoskop 1 einen Hauptkörper 2 sowie einen damit verbundenen starren Schaft 3, der an dem vom Hauptkörper 2 abgewandten distalen Ende 4 abgewinkelt ist. Aufgrund der starren Ausbildung des Schaftes, ist diese Abwinklung nicht änderbar.

Der starre Schaft 3 weist einen mit dem Hauptkörper 2 verbundenen ersten geradlinigen Abschnitt 5 auf, an den ein zweiter gebogener Abschnitt 6 anschließt. An den zweiten Abschnitt 6 schließt ein dritter Abschnitt 7 an, der das distale Ende 4 des Schaftes 3 bildet.

Der Hauptkörper 3 weist ein Hauptteil 8, mit dem der Schaft 3 verbunden ist, sowie einen Handgriff 9 auf.

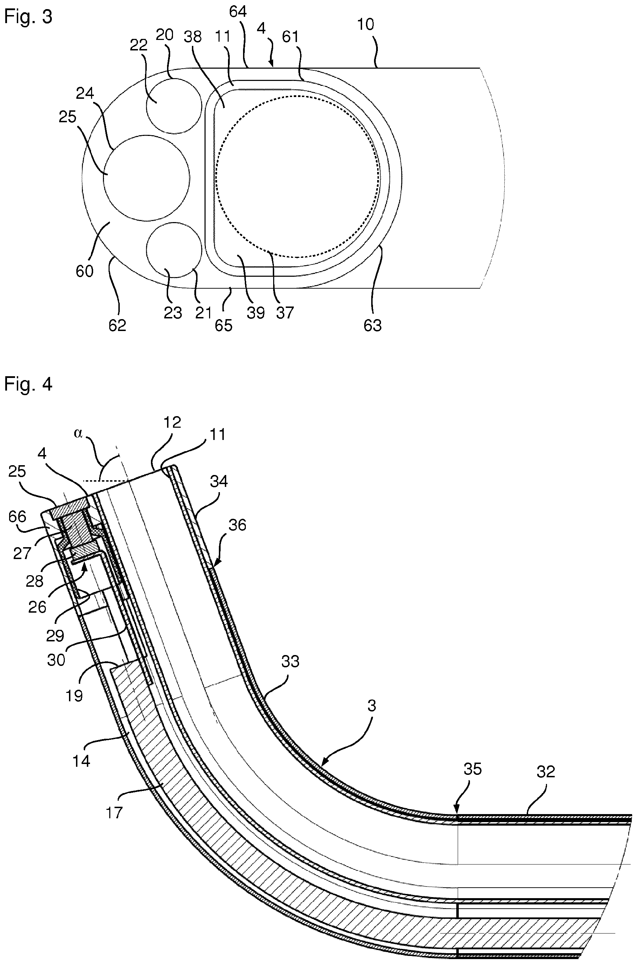

Wie am besten aus der Schnittdarstellung gemäß Fig. 2 ersichtlich ist, umfaßt der Schaft 3 ein Hüllrohr 10, das sich vom distalen Ende 4 bis hinein in das Hauptteil 8 erstreckt. In dem Hüllrohr 10 ist ein Instrumentenrohr 11 angeordnet, das sich bis zum distalen Ende 4 erstreckt und dort ein offenes Ende 12 aufweist. Das distale Ende des Hüllrohrs 10 ist zwar mit einer Endplatte 60 (Fig. 3) verschlossen. Für das Instrumentenrohr 11 weist die Endplatte 60 jedoch eine Öffnung 61 auf, so daß das offene Ende 12 von außen zugänglich ist. Das Instrumentenrohr 11, das einstückig und somit unterbrechungslos ausgebildet ist, erstreckt sich vom distalen Ende 4 durch das Hauptteil 8 des Hauptkörpers 2 hindurch und mündet in einem Anschlußstutzen 13. Über den Anschlußstutzen 13 kann ein Instrument in das Instrumentenrohr 11 eingeführt und bis zum distalen Ende 4 sowie darüber hinaus geschoben werden, da das Instrumentenrohr 11 am distalen Ende 4 das offene Ende 12 aufweist.

In dem Hüllrohr 10 ist ferner ein Optikkanal 14 vorgesehen, der, wie nachfolgend noch im Detail beschrieben wird, dazu dient, einen Bereich vor dem distalen Ende 4 beleuchten und ein Bild des beleuchteten Bereiches aufnehmen zu können.

Zur Beleuchtung des Bereiches vor dem distalen Ende 4 ist im Hauptteil 8 eine Leuchtdiode 15 in einer Bodenplatte 16 aus Aluminium positioniert, die Beleuchtungslicht abgibt, das in einen vor der Leuchtdiode 15 positionierten Lichtleiter 17 eingekoppelt wird. Dazu ist ein Ende des Lichtleiters 17 über einen Halter 18 direkt vor der Leuchtdiode 15 positioniert.

Der Lichtleiter 17 erstreckt sich von der Leuchtdiode 15 durch das Innere des Hauptteiles 8 in den Optikkanal 14 des Hüllrohres 10 und verläuft in diesem bis zum distalen Ende 4. Da die Endplatte 60 zwei Beleuchtungsöffnungen 20, 21 aufweist, wie am besten in Fig. 3 ersichtlich ist, in der eine Draufsicht des distalen Endes 4 gezeigt ist, teilt sich der Lichtleiter 17 nach Durchlaufen des zweiten gebogenen Abschnittes 6 des Schaftes 3 an einer Verzweigungsstelle 19 in zwei Lichtleiterabschnitte auf, die bis zu den beiden Beleuchtungsöffnungen 20, 21 laufen. In der Darstellung gemäß Fig. 2 laufen die beiden Lichtleiterabschnitte somit nach vorne und nach hinten aus der Zeichenebene gemäß Fig. 2 heraus. Daher endet in der Schnittdarstellung gemäß Fig. 2 der Lichtleiter 17 scheinbar an der Verzweigungsstelle 19.

Die beiden Beleuchtungsöffnungen 20, 21 sind jeweils mit einem Deckglas 22, 23 hermetisch dicht verschlossen (Fig. 3). Zwischen den beiden Beleuchtungsöffnungen 20 und 21 ist in der Endplatte 60 eine weitere Öffnung 24 im distalen Ende 4 ausgebildet, die wiederum mit einem Deckglas 25 hermetisch dicht verschlossen ist. Über diese Öffnung 24 erfolgt die Aufnahme des Bildes des beleuchteten Bereiches vor dem distalen Ende 4. Dazu ist hinter dem Deckglas 25 im Optikkanal 14 ein Aufnahmemodul 26 angeordnet. Dies ist besonders gut in der Schnittdarstellung von Fig. 4 zu sehen, in der das Detail A von Fig. 2 vergrößert dargestellt ist.

Das Aufnahmemodul 26 umfaßt hinter dem Deckglas 25 ein Objektiv 27 und einen Bildsensor 28 (der hier als CMOS-Sensor ausgebildet ist), die in einem Halter 29 sitzen, der dazu dient, das Objektiv 27 und den Bildsensor 28 hinter dem Deckglas 25 zu positionieren. Ferner ist in Fig. 4 noch schematisch ein Kabel 24 (hier Flachbandkabel) dargestellt, das mit dem Bildsensor 28 verbunden ist und vom Bildsensor 28 über den Optikkanal 14 bis zu einer entsprechenden Steuerelektronik, von der in Fig. 2 schematisch eine von zwei Platinen 31 eingezeichnet ist, im Handgriff 9 verläuft. Zur Vereinfachung der Darstellung endet das dargestellte Kabel 30 vor dem gebogenen zweiten Abschnitt 6.

Das Hüllrohr 10 ist hier aus drei Teilen 32, 33 und 34 gebildet, die an den Stellen 35 und 36 miteinander verschweißt sind. Das erste Teil 32 bildet den geraden Abschnitt 5. Das zweite Teil 35 bildet den gebogenen zweiten Abschnitt 6 und das dritte Teil 34 bildet zusammen mit dem zweiten Teil 33 den dritten Abschnitt 7 des Schaftes 3. Das zweite Teil 35 kann an seinen beiden Enden jeweils einen sich gerade erstreckenden Bereich aufweisen, der dann Bestandteil des ersten bzw. dritten Abschnitts 5, 7 ist. Der gebogene zweite Teil 33 ist hierbei so gebogen, daß ein Winkel α zwischen der Längsrichtung des dritten Abschnitts 7 und der Längsrichtung des ersten Abschnitts 5 70° beträgt. Bevorzugt ist der Winkel α größer als 0° und kleiner oder gleich 120°. Insbesondere ist der Winkel α größer oder gleich 10° und kleiner oder gleich 110°.

Durch diese mehrteilige Ausbildung des Hüllrohrs 10 läßt sich das erfindungsgemäße Endoskop 1 bzw. der Schaft 3 des erfindungsgemäßen Endoskops 1 leicht herstellen. So wird zur Herstellung des Endoskopes 1 das einstückige Instrumentenrohr 11 mit seinem distalen Ende durch das gebogene zweite Teil 33 hindurchgeschoben, bis der gekrümmte Abschnitt des Instrumentenrohrs 11 innen an dem entsprechend gekrümmten zweiten Teil 33 anliegt. Dann wird von der distalen Endseite das dritte Teil 34 aufgeschoben und von der proximalen Endseite das erste Teil 32. Danach wird das erste Teil 32 mit dem zweiten Teil 33 an der Stelle 35 verschweißt und das dritte Teil 34 mit dem zweiten Teil 33 an der Stelle 36 verschweißt.

Natürlich kann die Reihenfolge des Aufschiebens des dritten und ersten Teils 34, 32 auch umgekehrt sein. Ferner kann auch zuerst die Verschweißung an der Stelle 36 und dann an der Stelle 35 durchgeführt werden. Schließlich kann auch bereits nach Aufschieben des ersten oder dritten Teils 32, 34 die entsprechende Verschweißung durchgeführt werden und danach das verbleibende Teil (dritter oder erster Teil 34, 32) aufgeschoben und verschweißt werden.

Bevor das dritte Teil 34 auf das Instrumentenrohr 11 geschoben wird, können das Aufnahmemodul 26 sowie die Enden der Lichtleiterabschnitte des Lichtleiters 17 im dritten Teil 34 befestigt werden. Ferner können auch schon bereits die Deckgläser 22, 23 und 25 eingesetzt sein. Bevorzugt sind die Deckgläser 22, 23 und 25 verlötet, so daß sie in die entsprechende Öffnung 20, 21, 24 hermetisch dicht verschließen. Schließlich wird das Instrumentenrohr 11 im distalen Ende noch mit der entsprechenden Öffnung 61 in der Endplatte 60 verlötet oder verschweißt, so daß auch hier eine hermetisch dichte Verbindung in der Art vorliegt, daß zwar über das offene Ende 12 des Instrumentenrohrs 11 Flüssigkeit in das Instrumentenrohr 11 hinein- oder aus dem Instrumentenrohr 11 herausfließen kann. Jedoch besteht keine Verbindung vom Instrumentenrohr 11 bzw. vom Inneren des Instrumentenrohres 11 zum Optikkanal 14 oder zum Inneren des Hauptkörpers 2. Damit ist sowohl der Optikkanal 14 als auch der Hauptkörper 2 hermetisch dicht gegenüber der Umgebung verschlossen.

Wie insbesondere der Darstellung in Fig. 3 zu entnehmen ist, weist das Instrumentenrohr 11 einen im wesentlichen D-förmigen Querschnitt auf. Damit wird vorteilhaft erreicht, daß, wenn ein Instrument 37 im Instrumentenrohr 11 angeordnet ist, wie durch den gestrichelten Kreis in Fig. 3 angedeutet ist, noch zwei freie Bereiche 38, 39 im Instrumentenrohr 11 vorhanden sind, die zum Spülen und/oder Absaugen benutzt werden können. Über diese Bereiche 38, 39 kann eine Spülflüssigkeit dem Bereich vor dem distalen Ende 4 zugeführt werden. Ferner kann entsprechendes Material über diese Bereiche 38, 39 abgesaugt werden. Dazu kann, wie in Fig. 1 angedeutet ist, auf dem Anschlußstutzen 13 ein T-förmiges Anschlußelement 40 vorgesehen sein, das einerseits einen Zugang 41 für das einzuführende Instrument bereitstellt und andererseits einen Saug-/Spülanschluß 42 aufweist. Das Anschlußelement 40 ist nur in Fig. 1 und nicht in Fig. 2 eingezeichnet. Mit dieser Spülmöglichkeit können bei Verwendung des Endoskops 1 z.B. Verschmutzungen vor dem Deckglas 25, 22 und/oder 23 beseitigt werden, so daß dauerhaft gute Aufnahmebedingungen für das Aufnahmemodul 26 erzeugt und beibehalten werden können.

Der D-förmige Querschnitt des Instrumentenrohres 11 führt zu dem bereits beschriebenen Vorteil, daß bei eingesetztem Instrument 37 noch freie Bereich 38, 39 vorhanden sind, die als Spül- und/oder Absaugkanal verwendet werden können. Darüber hinaus ist der D-förmige Querschnitt äußerst kompakt, so daß auch der Querschnitt des Hüllrohrs 10 möglichst klein gehalten werden kann. Neben dem D-förmigen Querschnitt des Instrumentenrohrs 11 sind natürlich auch andere Querschnittsformen möglich, die bevorzugt so gewählt sind, daß neben einem kreisförmigen Querschnittsbereich (hier für das Instrument 37) noch zumindest ein freier Bereich (hier Bereich 38 und 39) vorliegt, der als Spül- und/oder Absaugkanal genutzt werden kann.

Damit das Hüllrohr 10 das Instrumentenrohr 11 aufnehmen kann, weist es keinen kreisförmigen Querschnitt auf, sondern einen von der Kreisform abweichenden Querschnitt, der beispielsweise als Doppel-D-Querschnitt bezeichnet werden kann. Der Querschnitt weist somit zwei gekrümmt verlaufende (hier halbkreisförmige) Enden 62, 63 auf, die durch geradlinige Seiten 64, 65 verbunden sind, die z.B. parallel zueinander verlaufen (Fig. 3). Um diese Querschnittsform des Hüllrohrs 10 in der perspektivischen Ansicht von Fig. 1 besser darstellen zu können, sind zwei sich in Längsrichtung des Hüllrohrs erstreckende Hilfslinien L1, L2 eingezeichnet, die den Übergang der beiden Enden 62, 63 zur geradlinigen Seite 65 verdeutlichen. Des weiteren ist noch eine Hilfslinie L3 eingezeichnet, die den Übergang vom gebogenen zweiten Abschnitt 6 zum sich geradlinig erstreckenden dritten Abschnitt 7 andeutet.

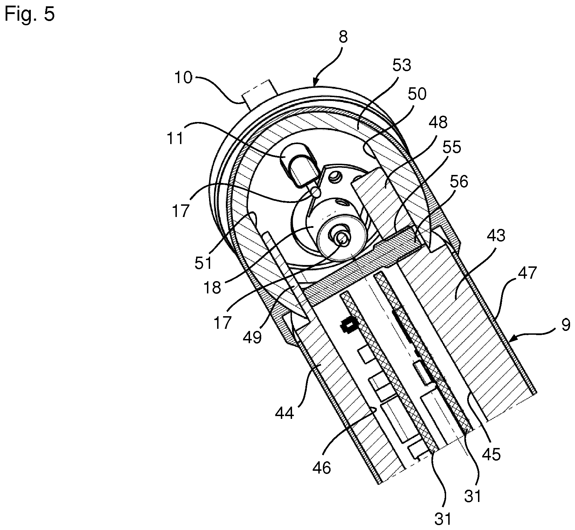

Die im Handgriff 9 angeordneten Platinen 31 dienen zur Steuerung des Bildsensors 28 sowie der Leuchtdiode 15. Die Platinen 31 sind im Handgriff 9 zwischen einem ersten und einem zweiten Wärmeleitkörper 43 und 44 angeordnet. Dies ist insbesondere in Fig. 5 ersichtlich, die eine Schnittdarstellung entlang der Schnittlinie B-B von Fig. 2 zeigt. Die Wärmeleitkörper 43, 44 weisen plane, einander zu weisende Innenseiten 45, 46 auf. Die Außenseiten der beiden Wärmeleitkörper 43 und 44 sind an die Innenkontur des hohlzylinderförmigen Wandungsabschnittes 47 des Handgriffs 9 angepaßt. Sowohl der erste als auch der zweite Wärmeleitkörper 43, 44 weisen jeweils einen vorstehenden Abschnitt 48, 49 auf, die in das Hauptteil 8 hineinstehen und jeweils an der entsprechenden Innenseite 50, 51 eines dritten Wärmeleitkörpers 53 anliegen, der mit seiner Außenseite an dem im wesentlichen hohlzylinderförmigen Wandungsabschnitt 54 des Hauptteils 8 anliegt. Der dritte Wärmeleitkörper 53 weist im Querschnitt im wesentlichen eine U-Form auf. Der dritte Wärmeleitkörper 53 steht in Kontakt mit dem Boden 16, wie in Fig. 2 gut ersichtlich ist.

Der vorstehende Abschnitt 48 des ersten Wärmeleitkörpers 43 weist ein Innengewinde 55 auf, in dem eine Schraube 56 eingeschraubt ist, deren dem Innengewinde 55 abgewandtes Ende gegen den vorstehenden Abschnitt 49 des zweiten Wärmeleitkörpers 44 drückt. Die Schraube 56 ist so in das Innengewinde 55 geschraubt, daß die beiden vorstehenden Abschnitte 48, 49 voneinander weg und somit gegen die Innenseiten 50, 51 des dritten Wärmeleitkörpers 53 gedrückt werden. Somit liegt zwischen den vorstehenden Abschnitten 48 und 49 und dem dritten Wärmeleitkörper 53 ein Flächenkontakt vor. Dies führt auch dazu, daß der dritte Wärmeleitkörper 53 gegen die Innenseite des hohlzylinderförmigen Wandungsabschnittes 54 gedrückt wird. Ferner führt das Spreizen der beiden vorstehenden Abschnitte 48, 49 mittels der Schraube 56 dazu, daß der erste und zweite Wärmeleitkörper 43 und 44 gut an dem hohlzylinderförmigen Wandungsabschnitt 47 anliegen.

Die Wärmeleitkörper 43, 44 und 53 sowie der Boden 16 sind aus Aluminium hergestellt, weisen eine hohe Wärmeleitfähigkeit bzw. eine höhere Wärmeleitfähigkeit als die Wandungsabschnitte 47 und 54 auf und dienen dazu, die beim Betrieb der Leuchtdiode 15 entstehende Wärme großflächig an die hohlzylinderförmigen Wandungsabschnitte 47 und 54 zu leiten und somit nach außen abzuführen. Die Wärmeleitkörper 43, 44 und 54 dienen somit zur Wärmespreizung. Die hohlzylinderförmigen Wandungsabschnitte 47 und 54 sind aus Edelstahl hergestellt und weisen eine deutlich geringere Wärmeleitfähigkeit als die Wärmeleitkörper 43, 44 und 53 auf. Aufgrund des großflächigen Kontaktes kann jedoch die Wärmeableitung sichergestellt werden.

Durch das Spreizen der beiden vorstehenden Abschnitte 48 und 49 ist der Kontakt zwischen den einzelnen Wärmeleitkörpern 43, 44 und 53 sowie der Kontakt der Wärmeleitkörper 43, 44, 53 zu den entsprechenden Wandungsabschnitten 47 und 54 durch die Kraftbeaufschlagung gewährleistet, die die Folge der Spreizung ist. Falls notwendig und/oder gewünscht, können die Wärmeleitkörper 43, 44 und 54 sowie der Boden 16 miteinander und/oder mit den entsprechenden Wandungsabschnitten 47 und 54 verklebt sein.

Am unteren Ende des Handgriffs 9 ist in Fig. 1 noch ein Kabel 57 eingezeichnet, das einerseits zur Stromversorgung der Leuchtdiode 15, der Platinen 31 sowie des Bildsensors 28 dienen. Andererseits werden über das Kabel 57 die Bilddaten der mittels des Bildsensors 28 aufgenommenen Bilder nach außen abgegeben. Dazu ist am Ende des Kabels 57 beispielsweise ein Stecker (nicht gezeigt) vorgesehen.

Das gesamte Endoskop 1 ist bis auf das Instrumentenrohr 11 hermetisch dicht gegenüber der Umgebung ausgebildet. Insbesondere der Optikkanal 14 und der Hauptkörper 2 sind gegenüber der Umgebung und dem Inneren des Instrumentenrohrs 11 hermetisch dicht. Dazu sind hier das Hüllrohr 10 sowie die Wandungsteile des Hauptkörpers aus Edelstahl hergestellt. Die abzudichtenden Verbindungsstellen sind bevorzugt verschweißt. Somit ist das Endoskop 1 autoklavierbar.

Da das Instrumentenrohr 11 einstückig und somit unterbrechungslos ausgebildet ist, läßt sich das Endoskop 1 in einem Autoklavierprozeß sehr gut reinigen und sterilisieren.

Durch die mehrteilige Ausbildung des Hüllrohrs 10 kann somit ein Endoskop mit einem starren Schaft 3, dessen Ende abgewinkelt ist, bereitgestellt werden, bei dem die Außenkontur des Schaftes 3 glatt ist und keine Kanten, Vertiefungen, Hinterschneidungen oder Vorsprünge aufweist, an denen sich leicht Schmutz ansammeln kann. Die Schweißstellen 35 und 36 können so abgeschliffen werden, daß keine Erhebung vorhanden ist, sondern eine glatte äußere Oberfläche des Hüllrohrs 10 vorliegt.

Der erste und zweite Teil 32 und 33 des Hüllrohrs sind bevorzugt aus einem Hohlrohrmaterial hergestellt. Dabei kann es sich insbesondere um ein durch Ziehen hergestelltes Rohrmaterial handeln. Ein solches Rohrmaterial läßt sich gut biegen. Der dritte Teil 36 ist bevorzugt durch spanende Bearbeitung aus einem Vollmaterial hergestellt. Damit läßt sich gut das distale Ende 4 und insbesondere die Endplatte 60 mit den Öffnungen 20, 21, 24 und 61 herstellen. Insbesondere können die Öffnungen 20, 21 und 24 so hergestellt werden, daß das jeweilige Deckglas 22, 23 und 25 im eingesetzten Zustand bündig mit der Oberseite der Oberplatte 60 ist. Des weiteren kann eine entsprechende Aufnahme 66 für das Optikmodul 26 ausgebildet sein (Fig. 4).

Das erfindungsgemäße Endoskop 1 ist insbesondere als Endoskop zur Anwendung im medizinischen Bereich ausgebildet. Ferner kann es als Endoskop 1 zur Anwendung im Hals-, Nasen-, Ohrenbereich dienen.

## Patentansprüche

1. Endoskop mit
einem Hauptkörper (2) und
einem sich vom Hauptkörper (2) erstreckenden starren Schaft (3), der einen sich geradlinig erstreckenden ersten Abschnitt (5), einen daran anschließenden gebogenen zweiten Abschnitt (6) und einen sich an den zweiten Abschnitt (6) anschließenden dritten Abschnitt (7), der ein distales Ende (4) des Schaftes (3) bildet, aufweist,
wobei der Schaft (3) zur Aufnahme eines Instrumentes (37) ein einstückig ausgebildetes Instrumentenrohr (11), das sich bis zum distalen Ende (4) des Schaftes (3) erstreckt und dort ein offenes Ende (12) aufweist, und ein Optikmodul (26), mittels dem ein Bild eines Bereiches vor dem distalen Ende (4) des Schaftes (3) aufnehmbar ist, aufweist,
und wobei ein sich vom Hauptkörper (2) bis zum distalen Ende (4) erstreckendes mehrteiliges Hüllrohr (10) vorgesehen ist, in dem das Instrumentenrohr (11) und das Optikmodul (26) angeordnet sind und das einen geradlinigen Teil (32) für den ersten Abschnitt (5) und einen mit dem geradlinigen Teil (32) verbundenen gebogenen Teil (33) für den zweiten Abschnitt (6) aufweist.

2. Endoskop nach Anspruch 1, bei dem das Hüllrohr (10) einen dritten Teil (34) aufweist, der das distale Ende (4) des Schaftes (3) bildet und mit dem gebogenen Teil (33) verbunden ist.

3. Endoskop ach Anspruch 2, bei dem das dritte Teil (34) einstückig ausgebildet ist und eine Endplatte (60) aufweist, die das dem gebogenen Teil abgewandte Ende verschließt, wobei in der Endplatte (60) eine Öffnung (61) für das Instrumentenrohr (11) und zumindest eine Öffnung (20, 21, 24) für das Optikmodul (61) ausgebildet ist.

4. Endoskop nach einem der obigen Ansprüche, bei dem die miteinander verbundenen Teile (32, 33, 34) des Hüllrohrs miteinander verschweißt sind.

5. Endoskop nach einem der obigen Ansprüche, bei dem der gebogene Teil (33) eine Biegung von größer als 0° und kleiner als 120° und insbesondere eine Biegung von größer oder gleich 10° und kleiner oder gleich 110° aufweist.

6. Endoskop nach einem der obigen Ansprüche, bei dem das Optikmodul (26) am distalen Ende (4) einen Bildsensor (28) aufweist.

7. Endoskop nach einem der obigen Ansprüche, bei dem das Instrumentenrohr (11) einen im wesentlichen D-förmigen Querschnitt aufweist.

8. Endoskop nach einem der obigen Ansprüche, bei dem das Hüllrohr (10) eine längliche Querschnittsform aufweist, die zwei gegenüberliegende abgerundete Enden (62, 63) sowie zwei geradlinig erstreckende, die Enden (62, 63) verbindende Seiten (64, 65) aufweist.

9. Endoskop nach einem der obigen Ansprüche, bei dem im Hauptkörper (2) eine Lichtquelle (15) angeordnet ist, die Licht abgibt, das über ein Lichtleitsystem (17) bis zum distalen Ende (4) geführt wird, um den vor dem distalen Ende (4) aufnehmbaren Bereich zu beleuchten.

10. Endoskop nach einem der obigen Ansprüche, bei dem die Lichtquelle (15) in direktem mechanischen Kontakt mit einem ersten Wärmeleitkörper (16) steht, der von der Lichtquelle (15) erzeugte Wärme zu einer Gehäusewandung (47, 54) des Hauptkörpers (2) leitet, wobei die Wärmeleitfähigkeit des ersten Wärmeleitkörpers (16) größer ist als die der Gehäusewandung (47, 54).

11. Endoskop nach Anspruch 10, bei dem in dem Hauptkörper (2) zumindest ein weiterer Wärmeleitkörper (43, 44) angeordnet ist, der in thermischem Kontakt mit dem ersten Wärmeleitkörper steht und mit einer Kraft beaufschlagt ist, die ihn gegen die Innenseite der Gehäusewandung (47, 54) drückt.

12. Endoskop nach einem der Ansprüche 10 oder 11, bei dem die thermische Kontaktierung des Wärmeleitkörpers mit der Gehäusewandung (47, 54) über eine reine Berührungskontaktierung ohne Wärmeleitkleber oder Wärmeleitpaste erzeugt ist.

13. Endoskop nach einem der obigen Ansprüche, bei dem der Hauptkörper (2) sowie der Schaft (3) mit Ausnahme des Instrumentenrohrs (11) gegenüber der Umgebung hermetisch abgedichtet und somit autoklavierbar sind.

14. Verfahren zur Herstellung eines Endoskops nach einem der obigen Ansprüche, bei dem folgende Schritte ausgeführt werden:
a) Einführen eines gebogenen Abschnittes des Instrumentenrohrs (11) in den gebogenen Teil (33),
b) Einführen eines geradlinigen Abschnittes des Instrumentenrohrs (11) in den geradlinigen Teil (32), und
c) Verbinden der beiden Teile (33, 32).

## Claims

1. Endoscope with
a main body (2) and
a rigid shaft (3), extending from the main body (2), which has a first section (5) which extends in a rectilinear manner, a curved second section (6) adjoining the latter and a third section (7), adjoining the second section (6), which third section forms a distal end (4) of the shaft (3),
wherein the shaft (3) has, for receiving an instrument (37), an instrument tube (11) formed as one piece which extends to the distal end (4) of the shaft (3) and has an open end (12) there, and an optics module (26) by means of which an image of an area in front of the distal end (4) of the shaft (3) can be recorded,
and wherein a multi-part cladding tube (10) extending from the main body (2) to the distal end (4) is provided, in which the instrument tube (11) and the optics module (26) are arranged and which has a rectilinear part (32) for the first section (5) and a curved part (33), connected to the rectilinear part (32), for the second section (6).

2. Endoscope according to claim 1, in which the cladding tube (10) has a third part (34) which forms the distal end (4) of the shaft (3) and is connected to the curved part (33).

3. Endoscope according to claim 2, in which the third part (34) is formed in one piece and has an end plate (60) which seals the end facing away from the curved part, wherein an opening (61) for the instrument tube (11) and at least one opening (20, 21, 24) for the optics module (61) are formed in the end plate (60).

4. Endoscope according to one of the above claims in which the parts (32, 33, 34) of the cladding tube which are connected to one another are welded to one another.

5. Endoscope according to one of the above claims in which the curved part (33) has a curvature of greater than 0° and smaller than 120° and in particular a curvature of greater than or equal to 10° and smaller than or equal to 110°.

6. Endoscope according to one of the above claims in which the optics module (26) has an image sensor (28) at the distal end (4).

7. Endoscope according to one of the above claims in which the instrument tube (11) substantially has a D-shaped cross-section.

8. Endoscope according to one of the above claims in which the cladding tube (10) has an elongated cross-section shape which has two rounded ends (62, 63) lying opposite one another as well as two sides (64, 65) which connect the ends (62, 63) and extend in a rectilinear manner.

9. Endoscope according to one of the above claims in which in the main body (2) a light source (15) is arranged which emits light which is guided via an optical fibre system (17) to the distal end (4) to illuminate the recordable area in front of the distal end (4).

10. Endoscope according to one of the above claims in which the light source (15) is in direct mechanical contact with a first heat-conducting body (16) which conducts heat generated by the light source (15) to a housing wall (47, 54) of the main body (2), wherein the thermal conductivity of the first heat-conducting body (16) is greater than that of the housing wall (47, 54).

11. Endoscope according to claim 10 in which in the main body (2) at least one further heat-conducting body (43, 44) is arranged which is in thermal contact with the first heat-conducting body and is loaded with a force which pushes it against the inside of the housing wall (47, 54).

12. Endoscope according to one of claims 10 or 11 in which the thermal contacting of the heat-conducting body with the housing wall (47, 54) is generated purely by touch without thermal adhesive or thermal conducting paste.

13. Endoscope according to one of the above claims in which the main body (2) and the shaft (3) except for the instrument tube (11) are hermetically sealed with respect to the surroundings and are thus autoclavable.

14. Process for producing an endoscope according to one of the above claims in which the following steps are carried out:
a) inserting a curved section of the instrument tube (11) into the curved part (33),
b) inserting a rectilinear section of the instrument tube (11) into the rectilinear part (32), and
c) connecting the two parts (33, 32).

## Revendications

1. Endoscope comprenant un corps principal (2) et
une tige rigide (3) s'étendant depuis le corps principal (2), qui présente une première portion (5) s'étendant en ligne droite, une deuxième portion courbe (6) s'y raccordant et une troisième portion (7) se raccordant à la deuxième portion (6), qui forme une extrémité distale (4) de la tige (3),
la tige (3) présentant, pour recevoir un instrument (37), un tube d'instrument (11) réalisé d'une seule pièce, qui s'étend jusqu'à l'extrémité distale (4) de la tige (3) et qui présente à cet endroit une extrémité ouverte (12), et un module optique (26) au moyen duquel une image d'une zone avant l'extrémité distale (4) de la tige (3) peut être enregistrée,
et un tube d'enveloppe (10) en plusieurs parties, s'étendant depuis le corps principal (2) jusqu'à l'extrémité distale (4) étant prévu, dans lequel le tube d'instrument (11) et le module optique (26) sont disposés, et qui présente une partie rectiligne (32) pour la première portion (5) et une partie courbe (33) pour la deuxième portion (6), connectée à la partie rectiligne (32).

2. Endoscope selon la revendication 1, dans lequel le tube d'enveloppe (10) présente une troisième partie (34) qui forme l'extrémité distale (4) de la tige (3) et qui est connectée à la partie courbe (33).

3. Endoscope selon la revendication 2, dans lequel la troisième partie (34) est réalisée d'une seule pièce et présente une plaque d'extrémité (60) qui ferme l'extrémité opposée à la partie courbe, une ouverture (61) pour le tube d'instrument (11) et au moins une ouverture (20, 21, 24) pour le module optique (61) étant réalisées dans la plaque d'extrémité (60).

4. Endoscope selon l'une quelconque des revendications précédentes, dans lequel les parties connectées les unes aux autres (32, 33, 34) du tube d'enveloppe sont soudées les unes aux autres.

5. Endoscope selon l'une quelconque des revendications précédentes, dans lequel la partie courbe (33) présente une courbure supérieure à 0° et inférieure à 120° et notamment une courbure supérieure ou égale à 10° et inférieure ou égale à 110°.

6. Endoscope selon l'une quelconque des revendications précédentes, dans lequel le module optique (26) présente, à l'extrémité distale (4), un capteur d'image (28).

7. Endoscope selon l'une quelconque des revendications précédentes, dans lequel le tube d'instrument (11) présente une section transversale essentiellement en forme de D.

8. Endoscope selon l'une quelconque des revendications précédentes, dans lequel le tube d'enveloppe (10) présente une forme en section transversale allongée qui présente deux extrémités opposées arrondies (62, 63) ainsi que deux côtés (64, 65) s'étendant en ligne droite, reliant les extrémités (62, 63).

9. Endoscope selon l'une quelconque des revendications précédentes, dans lequel une source de lumière (15) est disposée dans le corps principal (2), laquelle émet de la lumière qui est guidée par le biais d'un système de guide de lumière (17) jusqu'à l'extrémité distale (4), afin d'éclairer la zone pouvant être enregistrée avant l'extrémité distale (4).

10. Endoscope selon l'une quelconque des revendications précédentes, dans lequel la source de lumière (15) est en contact mécanique direct avec un premier corps thermoconducteur (16) qui conduit la chaleur générée par la source de lumière (15) jusqu'à une paroi de boîtier (47, 54) du corps principal (2), la conductibilité thermique du premier corps thermoconducteur (16) étant supérieure à celle de la paroi du boîtier (47, 54).

11. Endoscope selon la revendication 10, dans lequel au moins un corps thermoconducteur supplémentaire (43, 44) est disposé dans le corps principal (2), lequel est en contact thermique avec le premier corps thermoconducteur et est sollicité avec une force qui le presse contre le côté intérieur de la paroi de boîtier (47, 54).

12. Endoscope selon l'une quelconque des revendications 10 ou 11, dans lequel le contact thermique du corps thermoconducteur avec la paroi de boîtier (47, 54) est produit par le biais d'un simple contact physique sans colle thermoconductrice ni pâte thermoconductrice.

13. Endoscope selon l'une quelconque des revendications précédentes, dans lequel le corps principal (2) ainsi que la tige (3), à l'exception du tube d'instrument (11), sont étanchéifiés hermétiquement vis-à-vis de l'environnement et peuvent ainsi être traités en autoclave.

14. Procédé de fabrication d'un endoscope selon l'une quelconque des revendications précédentes, dans lequel les étapes suivantes sont mises en oeuvre :
a) introduction d'une portion courbe du tube d'instrument (11) dans la partie courbe (33),
b) introduction d'une portion rectiligne du tube d'instrument (11) dans la partie rectiligne (32), et
c) assemblage des deux parties (33, 32).
